# EUROPEAN PATENT APPLICATION

(11) **EP 2 027 781 A1**
(43) Date of publication of application: **25.02.2009**
(21) Application number: 07016336.5
(22) Date of filing: 21.08.2007
(51) Int. Cl.: A23L 1/211, A23L 1/30

(54) **Method of removal of bitter taste from olive juice extract**

(71) Applicant: DSM IP Assets B.V., 6411 TE Heerlen (NL)
(72) Inventor: The designation of the inventor has not yet been filed
(74) Representative: Pressner, Dietmar

(57) **Abstract**

Olive juice is de-bittered by raising the pH to about 9 while increasing the temperature. Under these conditions, bitter tasting compounds are hydrolyzed, and the resulting juice has both an increased hydroxytyrosol content, and is non-bitter. This extract can be further processed, or used as a component of a nutritional composition or nutritional supplement.

## Description

### FIELD OF THE INVENTION

This invention relates to olive juice extracts which no longer have a bitter taste, and to methods of making these extracts, and to the novel extracts so produced. This invention also relates to nutritional and food products containing these extracts.

### BACKGROUND OF THE INVENTION

The medicinal benefits of olive oil and olive extracts is widely being recognized. To make olive oil, the olive fruits are ground into a paste. Pressure is applied to the paste to separate the oil from the ground fruit. In addition to providing olive oil, the pressing also releases the water based content of the olive fruit, which contains many water soluble phytochemicals. This water is known by a number of names, including "vegetation water, olive juice, and olive waste water". Interestingly, while olive juice and its disposal can be a problem for the olive oil producer, it can also be a desirable rich source of phenolic compounds, which can have beneficial nutritional properties. In its natural state, olive juice is rather dilute, so olive juice extracts have been made.

In the past, methods concentrate the olive juice involved time consuming incubation, filtration and/or centrifugation, and spray-drying steps. Another problem is that the usability of dried or liquid olive juice in food or dietary supplements is limited due to the smell, bitterness, and turbidity of the olive juice, as well as the low content of hydroxytyrosol, one of the active polyphenols.

It would therefore be desirable to develop a better method for producing an all-natural, hydroxytyrosol-rich, non-bitter olive juice extract which is efficient and cost-effective.

### DESCRIPTION OF THE INVENTION

In accordance with this invention, a new process for making an all-natural, non bitter olive juice extract is presented. Also as part of this invention are novel nutritional compositions containing this extracted olive juice, and to the novel olive juice extract, and olive juice derivatives in powdered form.

Thus this invention relates to a method for removing the bitter taste from olive juice resulting from the presence of bitter tasting compounds, comprising maintaining the juice at an elevated pH and temperature for a time sufficient for the bitter tasting compounds to hydrolyze, and thereby lose their bitter taste.

A typical olive will contain approximately 50% water, 22% oil, 19% carbohydrates, 6% cellulose, 2% proteins, and oleuropein and hydroxytyrosol (combined) 0.2%. It should be appreciated that the exact makeup of the fruit (and its subsequent extract) can vary according to the cultivar of olive, the time of harvest, and even growing conditions.

As used throughout the specification and claims, the following definitions apply:
"HT" means hydroxytyrosol.
"Olive Juice", "Olive Waste Water" and "Vegetation Water" are terms all intended to be used interchangeably. They refer to the water phase produced when crushing olive fruits during olive oil production. It is a slurry with a complex mixture of carbohydrates along with the compounds of interest, such as HT and oleuropein (which contains bound HT, and which may be subsequently broken down to yield HT).

Contrary to literature reports, (see, e.g. Briante et al, 2002, J. Biotechn. 93: 109-119, and Soler-Rivas et al 2000, JSci Food Agric 80:1013-1023) it has been found, in accordance with this invention, that the unknown compounds which impart a bitter taste to the olive juice are neither hydroxytyrosol nor oleuropin. While not wishing to be bound by theory, they may contain a labile phenolic ester group. Regardless of the identity of the bitter compounds, they are very sensitive to base, and are not stable at higher pH.

Any form of olive juice may be used as a starting material for this processes of this invention, although minor variations of the processes may be required for optimal results. Fresh juice typically containing about 85-90% water and 10-15% solid matter can be purchased and used as a starting material in the processes of this invention. Other forms of commercially available olive juice which have been filtered by the supplier to remove some or all of the solids, are also appropriate starting materials, as well. Also appropriate is olive juice which is purchased in a concentrated form (for example, 3-4 X), or concentrated prior to use by simple evaporating techniques known in the art. In other cases, the olive juice may be conveniently purchased in a stabilized, freeze dried form where citic acid has been added to stabilize it. Dried olive juice contains about 60% carbohydrates, 10% fibers, 10 % fat, and 6% polyphenols (this includes approximately 2% hydroxytyrosol, 0.2% tyrosol and other polyphenols), and organic acids, proteins and minerals. Generally, for economic reasons, a concentrate is preferred, but the methods described will work with any of the juices.

It has been found, in accordance with one aspect of this invention, that the bitterness associated with the taste of olive juice can be easily remove by subjecting the juice to heat treatment at an elevated pH for a sufficient time to remove all bitter taste. In general, the step of removing bitterness will be among the first steps in a multi-step process to make an olive juice extract. In preferred methods, this step is performed early in the overall process of making an olive juice extract. However, this step may be perform at any time during the extraction process which is deemed convenient.

The elevated temperature can exist over a wide range, i.e, from about 20 degrees C up to about 100 degrees C. The actual temperature is not especially critical. Preferred temperature ranges are from about 60 to about 95 degrees C, and a more preferred temperature is about 80 degrees C.

Along with the increase in the temperature, the pH of the juice should be adjusted to a higher pH than is originally present in the untreated olive juice. The adjusted pH should be in a range of about 6 to about 12, with the preferred pH being from about 7 to about 10, and even more preferably, 8 to about 9. A pH above about pH 10 needs careful exclusion of oxygen, because at this pH, the HT rapidly degrades, hence a pH≤ 9 being preferred Any basic compound capable of adjusting pH can be used. Preferred compounds are NaOH, KOH, and mixtures thereof. Generally, the amount needed will vary from batch to batch, but a typical amount needed will be approximately 10-15% by weight based on the weight of the batch.

Under the conditions of the elevated temperature and pH, complex compounds which contain bound HT will break down, liberating HT. Thus, after the reactions are substantially complete, the juice will contain a higher amount of HT than the starting material.

During the time that the reactions are taking place, the pH may change, and readjustment(s) may be required to maintain the desired pH. It is preferred to continually adjust the pH during the time of the reaction. One way of determining that the hydrolysis reactions are substantially complete is that the pH will remain relatively stable (i.e. relatively unchanged) for at least about ten minutes. The amount of time it will take for a batch to reach the point of stability for about 10 minutes can vary greatly depending on the individual composition of the batch.

At this point, not only is the amount of HT increased (assuming that the starting material was bitter) the olive juice no longer is bitter. Thus a further aspect of this invention is an olive juice extract which, in comparison to raw olive juice, comprises an increased amount of HT, and is non-bitter. The processor can use this non-bitter composition in any way desired. For example, it can be directly incorporated into a nutritional composition, for example a food or beverage composition. The food can be suitable for human consumption, or can be an animal feed. Alternatively it can be made into a nutritional supplement, for example it can be formulatd into capsules, tablets, or the like using known methods. Alternatively the composition can be used in cosmetic compositions.

In another embodiment of the invention, the non-bitter extract is spray dried or freeze dried using conventional techniques to form a powder derivative, and then incorporated into a final product.. Optionally, stabilizers and the like may be added to the juice prioir to the drying step. The resulting powder it can be directly incorporated into a nutritional composition, for example a food or beverage composition. The food can be suitable for human consumption, or can be an animal feed. Alternatively it can be made into a nutritional supplement, for example it can be formulatd into capsules, tablets, or the like using known methods. Alternatively the composition can be used in cosmetic compositions.

Alternatively either the juice or powdered derivative can be made into a nutritional supplement, for example it can be formulatd into capsules, tablets, or the like using known methods.

In another aspect of this invention, the olive juice extract so made is the starting material for further processing, such as that described in co-pending patent application number ____, [Attorney Docket Number 26254], filed herewith.

The following non-limiting examples are presented to better illustrate the invention.

### Examples

### Example 1

### Starting Material

Unless otherwise stated, all %s given refer to weight percent.

Starting material was HIDROX 6% (from CreAgri, Haywood, CA Lot 6022406002. This is freeze-dried olive juice which has been stabilized with citric acid. Its content is: (in weight %):

| | |
|---|---|
| Carbohydrates | Approx. 60% |
| Lipids (olive oil) | Approx. 15% |
| Fibers | Approx, 10% |
| Hydroxytyrosol | 2.1% |
| Water | Approx. 2% |

Appearance:

| | |
|---|---|
| Solubility in water: | a turbid, fine suspension |
| Taste | bitter |
| Color | beige-brown powder |

### EXAMPLE 2

### Removal of bitterness

In a reaction flask a solution of 100 g HIDROX 6% (from Example 1 which had HT 2.1 %, and tyrosol 0.24%) and 80 ml water was prepared. The mixture was stirred under a nitrogen blanket, and warmed up to 80 degrees C. The mixture was adjusted to pH = 9.0 and stirred for 30 minutes at 80 degrees C at constant pH with continuous addition of a total of 38 ml NaOH (10 mo/l), for a total of 380 mmole. It is noted that fresh olive juice will require less NaOH as the dried juice has been stabilized with approximately 1% citric acid, so its starting pH is lower than fresh juice.

The suspension was no longer not bitter. It can be used as such, or concentrated to a dried powder in known manner e.g. freeze dried or spray dried.

The content of hydroxytyrosol increased by the base treatment to approximately 130%.

### Example 3

The following Table 1 shows results, performed essentially as in Example 2, except for pH and/or atmospheric adjustment adjustment, noted in the table. At 80 degrees C, the bitterness disappears in approximately 30 minutes at pH 8, and in aproximately 10 minutes at pH 9, without changing much of the olive smell. At a higher pH (≥10), the smell of the olive juice changes to an unpleasant woody odor. Oxygen destroys HT increasingly rapidly above pH 9.

In the Table 1 below, color is indicated as: ++ =increased; + = standard; (=) = decreased; and (-) = not determined. Dilution used for testing bitterness was approximately 1 mg/ml tap water. The HT wt % has been normalized to dried olive juice.??

**TABLE 1**

| **Hydrolysis of Bitter Compounds at Various pH** | | | | | |
|---|---|---|---|---|---|
| **No.** | **pH** | **Atmosphere** | **Time (h)** | **Bitterness** | **HT w%** |
| Ref | 3.5 | N2 | 1 | + | 2.1 |
| B1 | 6.0 | N2 | 0.25 | + | n.d. |
| B2 | 7.0 | N2 | 0.75 | (+) | 2.8 |
| A | 8.0 | N2 | 0.25 | - | 2.9 |
| D | 8.0 | O2(air) | 0.25 | - | 2.9 |
| E | 9.0 | N2 | 1 | - | 3.3 |
| C | 10.0 | N2 | 0.25 | - | 3.4 |
| G | 11.0 | N2 | 0.75 | - | 3.0 |

## Claims

1. A method for removing the bitter taste from olive juice resulting from the presence of bitter tasting compounds, comprising maintaining the juice at an elevated pH and temperature for a time sufficient for the bitter tasting compounds to hydrolyze, and thereby loose their bitter taste.

2. A method according to Claim 1 wherein the elevated temperature is from about 20 degrees to 100 degrees C, and the pH is from about 6 to about 12.

3. A method according to Claim 2 wherein the elevated temperature is maintained until the pH remains unchanged for at least about 10 minutes.

4. A method according to Claim 2 wherein the pH is adjusted by addition of a base.

5. A method according to Claim 4 wherein the base is NaOH or KOH.or a mixture thereof.

6. A method according to Claim 4 wherein the temperature is about 70- 90 degrees C and the pH is about 8-9.

7. A non-bitter hydroxytyrosol enriched olive juice produced by any of the processes of Claims 1-6.

8. A method of making a powdered non-bitter hydroxytyrosol enriched olive juice derivative comprising producing the olive juice of Claim 7 and
spray drying or freeze drying to produce the powdered olive juice derivative.

9. A method according to Claim 8 additionally comprising adding a stabilizer to the olive juice prior to the drying step.

10. The powdered olive juice derivative produced by the method of Claim 8 or Claim 9.

11. A nutritional composition comprising the olive juice extract of Claim 7 or 10.

12. A nutritional supplement comprising the olive juice extract of Claim 7 or 10.

13. A feed composition or supplement comprising the olive juice extract of Claim 7 or 10.

14. A cosmetic composition or supplement comprising the olive juice extract of Claim 7 or 10.
